# EUROPEAN PATENT SPECIFICATION

(11) **EP 3 886 934 B1**
(45) Date of publication and mention of the grant of the patent: **03.12.2025**
(21) Application number: 19874776.8
(22) Date of filing: 28.11.2019
(51) Int. Cl.: A61M 1/00, A61F 13/00, A61M 3/02, A61F 13/05

(54) **WOUND-DRESSING CONDITIONING DEVICE**
WUNDVERSORGUNGSKONDITIONIERUNGSVORRICHTUNG
DISPOSITIF DE CONDITIONNEMENT DE PANSEMENT

(30) Priority: 29.11.2018 GB 201819440
(43) Date of publication of application: 06.10.2021
(73) Proprietor: Nexa Medical Limited, Bournemouth, Dorset BH22 8TR (GB)
(72) Inventor: HARDMAN, Ian, Bournemouth BH9 3PS (GB); HEATON, Keith, Bournemouth BH22 8TR (GB)
(74) Representative: Hocking, Adrian Niall
(86) International application number: PCT/GB2019/053370
(87) International publication number: WO 2020/109804

(56) References cited:
- WO-A1-2016/010792
- WO-A1-2017/077312
- GB-A- 2 511 523
- US-A1- 2007 293 830
- US-A1- 2011 313 343

## Description

The invention relates to a wound-dressing conditioning device.

Applying a reduced or negative pressure at a wound site is beneficial to wound healing, such as by removing infectious material and/or exudates. This type of therapy is typically referred to as `negative pressure wound therapy" or "NPWT".

The application of saline, cleansing fluid or an active wound treatment substance to the wound, for instance to reduce the bacterial load, is known as an "irrigation" or a "lavage". If the treatment solution is left for a period of time at the wound to take effect, this is known as "instillation". The combination of NPWT with irrigation or instillation has been shown to further promote wound healing.

At present, combining NPWT with irrigation or instillation is complicated due to the requirements of pumping fluid into the wound at a controlled rate, removing the fluid into a separate waste container and applying a constant negative pressure at the wound site.

Current solutions are expensive to produce, require significant training to use as well as requiring ongoing maintenance. Disadvantages include the need to have separate pumps, one for the fluid instillation and one for removing the fluid, or complex valve arrangements. Separate pumps, often used with rigid containers, result in more equipment being needed and thus, a larger space requirement. In the case of the single vacuum pump, downstream of the wound, it is difficult to measure accurately the fluid entering the wound. This increases the risk of over filling the wound with fluid and breaching the seal around the periphery of the wound. Electronic negative pressure monitoring is also required to ensure excessive negative pressure is not present at the wound site. Furthermore, pumps which come into contact with the wound waste and/or irrigation or instillation fluids may need extensive cleaning or need to be disposed of, which increases the costs. US20070293830A discloses an apparatus for cleaning wounds.

The present invention seeks to provide a solution to these problems.

According to an aspect of the present invention, there is provided a wound-dressing conditioning device for conditioning a wound dressing, the device comprising: a treatment-fluid flow path having a treatment-fluid inlet for fluidly communicating with a treatment-fluid source, and a treatment-fluid outlet for fluidly communicating with the wound dressing; a waste-fluid flow path having a waste-fluid inlet for fluidly communicating with a wound dressing, and a waste-fluid outlet for fluidly communicating with a waste-fluid container, and a pumping means at or adjacent to the treatment-fluid flow path and the waste-fluid flow path, the in-use pumping means having a treatment-fluid-application condition for pumping treatment fluid from the treatment-fluid inlet to the treatment-fluid outlet, a waste-fluid-removal condition for pumping waste fluid from the waste-fluid inlet to the waste-fluid outlet, and a negative-pressure condition for applying negative pressure to the wound dressing; a peristaltic pump having a controller to control the treatment-fluid-application condition, and a monitoring means for monitoring a volume of treatment fluid pumped to the treatment-fluid outlet during the treatment-fluid-application condition, wherein the waste-fluid flow path is entirely or substantially entirely defined by a waste tubing-section and the treatment-fluid flow path is entirely or substantially entirely defined by a treatment tubing-section, the waste tubing-section and the treatment tubing-section being separate, and the positive pressure regulator is directly fluidly communicable with the waste-fluid flow path, characterised in that the peristaltic pump has one pump head, the treatment tubing section and the waste tubing section being positioned in opposing orientations on the pump head.

The invention provides a user with a means to promote wound healing by cleansing a wound with treatment fluids to perform an irrigation or a lavage; and applying negative pressure wound therapy, simultaneously or one after the other. Pumping the treatment fluid directly, rather than passively allowing the treatment fluid to be introduced to the wound dressing, allows for monitoring and control over the volume of the treatment fluid applied. The ability to monitor and control the treatment fluid pumped to the wound dressing prevents or limits over pressurisation of the wound dressing. This in turn prevents or limits rupturing, unsticking and/or delamination of the wound dressing.

Separate tubing-sections or conduit portions allow for treatment fluids to be pumped to the wound and waste fluids to be removed from the wound separately or simultaneously if required. Each tubing-section can also be easily replaced without requiring the other or all tubing-sections to be replaced. Flow within each tubing-section may be controlled by separate pumps.

A peristaltic pump allows a known volume to be pumped with every pump rotation. By pumping fluid without contacting said fluid, the peristaltic pump is re-usable without requiring any or any substantial cleaning and need not be disposed of, thereby reducing cleaning effort, staff time, equipment requirements, and thereby costs. A peristaltic pump may also function as a seal between the wound dressing and the treatment fluid source and/or between the wound dressing and the waste-fluid container.

Flow within each tubing-section is controllable by a single pump having a single head, thereby reducing the number of pumps required. Furthermore, a single pump, having one head results in the volume of treatment fluid pumped to the wound equalling the volume of waste fluid being removed. This results in positive pressure in the treatment tubing-section and negative pressure in the waste tubing-section. If simultaneous, net neutral or substantially neutral pressure may result at the wound dressing. Additionally, there may be no requirement for a separate and/or complicated pressure monitor.

Preferably, the pump has a pumping cycle during which a known volume of fluid is pumped, and the number of pumping cycles carried out by the pumping means is determinable by the monitoring means to determine the volume of treatment fluid pumped to the treatment-fluid outlet. Beneficially, the controller may be linked to the monitoring means, the treatment-fluid-application condition being terminable by the controller when the monitoring means determines a predetermined volume of treatment fluid has been pumped. The pumping means associated with the controller and the monitoring means allows a user to more accurately control and measure the volume, dose or flow rate of medicine being administered at the wound. The advantage is an increased accuracy in the dose of the active substance being administered to a patient, and thereby, a reduced risk of overdosing. A better volume-control also reduces the risk of a wound dressing, bandage or other administration means leaking, becoming overfilled and/or accidentally detached.

Preferably, the wound-dressing conditioning device further comprises a wound dressing connectable to the treatment-fluid outlet and the waste-fluid inlet. The wound dressing improves the administration of treatment fluids, particularly by ensuring unifonn or substantially unifonn distribution of fluids to the wound. A wound dressing also prevents or inhibits leakage, reducing costs and cleaning efforts. Additionally, it may provide a barrier to prevent or inhibit infection and/or undesirable foreign bodies from entering the wound. In this case, the wound dressing also provides the ability to apply negative pressure wound therapy by providing a sealable local environment over the wound.

Preferably, the wound dressing may include a wound filler and a visual indicator, the visual indicator having at least one visual difference to the wound filler and being positioned at, in or on the wound filler, the wound filler being contractible by negative pressure so as to at least in part expose the visual indicator and/or expandable by positive pressure so as to at least in part cover the visual indicator.

Beneficially, the wound filler may comprise an aperture which extends at least part-way therethrough, the indicator being positioned in said aperture, and the wound filler being contractible by negative pressure so as to enlarge an opening of said aperture and/or expandable by positive pressure so as to at least in part close an opening of said aperture.

Advantageously, the wound filler may be more compressible and/or expandable than the visual indicator.

Additionally, the wound-dressing conditioning device may further comprise a wound-dressing connector for connecting the wound dressing to the treatment-fluid outlet and/or the waste-fluid inlet. A wound dressing connector allows for the quick and efficient connection of a wound dressing with the flow paths.

Tubing confines fluid to the fluid flow path and provides a compressible volume so as to allow peristaltic pumping. The wound dressing being detachably connectable with tubing, allows for modularity and ease of replacement. For example, changing the treatment fluid to be applied can be done without necessarily requiring the wound dressing to be changed and vice-versa.

Preferably, the wound-dressing conditioning device further comprises a negative pressure regulator in the waste-fluid flow path and/or the treatment-fluid flow path, the negative pressure regulator comprising a valve configured to be automatically openable to the exterior of the flow path to increase pressure at the wound dressing and automatically closeable to maintain pressure. The negative pressure regulator prevents or limits a medically unacceptably high vacuum being applied to the wound. This prevents or limits damage to the wound. The valve is preferably a mechanical valve.

Advantageously, the wound-dressing conditioning device may further comprise a positive pressure regulator in the treatment-fluid flow path and/or the waste-fluid flow path, the positive pressure regulator comprising a valve configured to be automatically openable to the exterior of the flow path to decrease pressure at the wound dressing, and automatically closeable to maintain pressure. The positive pressure regulator prevents or limits pressure build up in the wound dressing and/or tubing. This in turn prevents or limits the risk of rupture of tubing or wound dressing. The valve is preferably a mechanical valve.

Furthermore, the positive pressure regulator may include a vent conduit having a pressure relief valve, the vent conduit being fluidly communicable with the waste-fluid container and/or the fluid treatment source to vent excess treatment fluid. This feature reduces the risk of leakage and overfilling the wound dressing. Furthermore, if the excess treatment fluid is returned to the fluid treatment source, prior to mixing with the wound exudates, the excess treatment fluid may be stored for future use, thereby reducing wastage and costs.

The invention will now be more particularly described, by way of example only, with reference to the accompanying drawings, in which:
Figure 1 shows a first embodiment of an in-use wound-dressing conditioning device not in accordance with the invention having a pumping means in a treatment-fluid-application condition and block arrows indicating a direction of the treatment-fluid flow path;
Figure 2 shows the wound-dressing conditioning device of Figure 1 with the pumping means being in a waste-fluid removal condition and block arrows indicating a direction of the waste-fluid flow path;
Figure 3 shows an enlarged view of a negative pressure regulator of the wound-dressing conditioning device of Figure 1, with a positive pressure regulator omitted for clarity;
Figure 4 shows an enlarged view of a positive pressure regulator of the wound-dressing conditioning device of Figure 1, with the negative pressure regulator omitted for clarity;
Figure 5 shows an upstream end portion of a second embodiment of an in-use wound-dressing conditioning device not in accordance with the invention having a pumping means in a treatment-fluid-application condition and block arrows indicating a direction of the treatment-fluid flow path;
Figure 6 shows the upstream end portion of Figure 5 with the pumping means being in a waste-fluid removal condition and block arrows indicating a direction of the waste-fluid flow path;
Figure 7 shows a third embodiment of the wound-dressing conditioning device not in accordance with the invention;
Figure 8 shows a pump of the wound-dressing conditioning device of Figure 7 with first and second pump heads, both pump heads being operable by a motor of the pump;
Figure 9 shows the pump of Figure 8 with the first pump head operable by the motor and the second pump head inoperable by the motor,
Figure 10 shows the pump of Figure 8 with the second pump head operable by the motor and the first pump head disengaged from the motor.
Figure 11 shows a fourth embodiment of the wound-dressing conditioning device in accordance with the first aspect of the invention;
Figure 12 shows the positive pressure regulator of Figure 4 connected to a treatment-fluid source;
Figure 13 shows the positive pressure regulator of Figure 4 connected to a waste-fluid container,
Figure 14 shows the positive pressure regulator of Figure 4 connected to a waste-fluid flow path and the negative pressure regulator of Figure 3;
Figure 15 shows an embodiment of a wound dressing not in accordance with the invention in a high pressure, positive pressure, or atmospheric pressure state, including a wound filler and a visual indicator, their differing visual appearances represented by differing cross-hatching; and
Figure 16 shows the wound dressing of Figure 15 in a low pressure or negative pressure state.

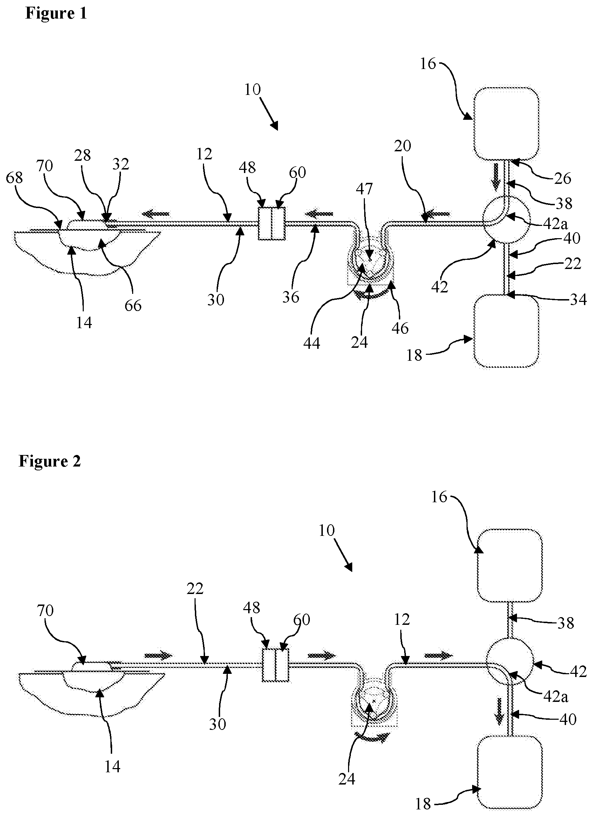

Referring firstly to Figures 1 and 2 there is shown a wound-conditioning apparatus 10 comprising a wound-dressing conditioning device 12, a wound dressing 14, a treatment-fluid source 16 and a waste container 18.

The wound-dressing conditioning device 12 comprises a treatment-fluid flow path 20, a waste-fluid flow path 22 and a pumping means 24 or pump. The treatment-fluid flow path 20 in use allows for the application of positive pressure to the wound dressing 14. The positive pressure preferably enables the application of treatment fluid to the wound via the wound dressing 14. The waste-fluid flow path 22 allows for the application of negative pressure to the wound via the wound dressing 14. The negative pressure preferably enables removal of waste fluids from the wound and/or the wound dressing 14 and applying a vacuum or negative pressure to the wound dressing 14.

The treatment-fluid flow path 20, or instil line, has a treatment-fluid inlet 26 in fluid communication with the treatment-fluid source 16 and a treatment-fluid outlet 28 in fluid communication with the wound dressing 14. Between the treatment-fluid inlet 26 and treatment-fluid outlet 28, the treatment-fluid flow path 20 is defined by or contained within tubing 30, a tube, a conduit or a pipe. The tubing 30 is preferably at least in part flexible or pliantly flexible. Additionally, the tubing 30 preferably has a round or circular cross-section and may comprise polymer or plastics, particularly a bio-compatible polymer such as Polyvinyl chloride PVC, Thermoplastic polyurethane TPU or silicone. However, the tubing could also be flat, oval, circular or non-circular in profile.

The waste-fluid flow path 22, or vacuum line, preferably comprises a waste-fluid inlet 32, fluidly communicative with the wound dressing 14, and a waste-fluid outlet 34, fluidly communicative with a waste container 18. The waste-fluid flow path 22 is defined between its inlet and outlet 32, 34 by tubing 30 in a similar or identical way to the treatment-fluid flow path 20.

The treatment-fluid outlet 28 and the waste-fluid inlet 32 are defined by the same opening in the tubing 30, and the treatment-fluid inlet 26 and the waste-fluid outlet 34 are defined by different openings of the tubing 30. The treatment-fluid flow path 20 and the waste-fluid flow path 22 at least in part overlie, overlap, and/or are at least in part defined by or contained within the same extent, longitudinal extent, portion or section of tubing 36. Said same extent of tubing 36 may be defined as a dual tubing-section 36, and although described as a dual tubing-section it will be appreciated that the dual tubing-section may not necessarily comprise a dual lumen or two lumina At least part of the treatment-fluid flow path 20 and the waste-fluid flow path 22 are defined by separate extents of tubing 30; the treatment tubing-section 38 and the waste tubing-section 40 respectively.

The device 12 preferably further comprises a selector valve 42 to selectably alternate between fluidly communicating the dual tubing-section 36 with the treatment tubing-section 38, and therefore the treatment-fluid inlet 26, and the waste tubing-section 40, and therefore the waste-fluid outlet 34. Here the device 12 includes a rotary selector valve 42 at a three-way intersection between the dual tubing-section 36, the treatment tubing-section 38 and the waste tubing-section 40. The rotary selector valve 42 preferably includes a sealed head with at least one fluid path 42a that can be rotated to interengage the dual tubing-section 36 and the treatment tubing-section 38 or the waste tubing-section 40. The fluid path 42a could be driven by a suitable motor such as a stepper motor or a D.C motor with an encoder. The motor may be electronically controlled by the user or via a controller. Alternatively, the selector valve 42 could be manually actuated. The device may have the option of attaching at least one further container, such as a second treatment container or an overflow waste container.

The pumping means 24, or pumping element, is on, at or adjacent to the treatment-fluid flow path 20 and the waste-fluid flow path 22. Here this is achieved via positioning the pumping means 24 on, at or adjacent to the dual tubing-section 36. The pumping means 24 has a treatment-fluid-application condition, for pumping treatment fluid from the treatment-fluid inlet 26 to the treatment-fluid outlet 28, and a waste-fluid-removal condition, for pumping waste fluid from the waste-fluid inlet 32 to the waste-fluid outlet 34. Preferably the pumping means 24 further has a negative pressure condition for applying negative pressure or a vacuum to the wound and/or the wound dressing 14.

The pumping means 24 is a pump which is, preferably but not necessarily, reversible so that so that the pump 24 can pump fluid along the dual tubing-section 36 in a direction towards the wound dressing 14 and away from the wound dressing 14. Pumping fluid towards the wound dressing 14 is required for the treatment-fluid-application condition, thereby applying positive pressure; and pumping fluid away from the wound dressing 14 is required for the waste-fluid removal and negative-pressure conditions. Here the pump 24 is a peristaltic pump, and therefore acts on the flow path but external to the tubing 30.

The pump 24 acts on the treatment-fluid flow path 20 and waste-fluid flow path 22 between the treatment-fluid inlet 26 and the treatment-fluid outlet 28, and between the waste-fluid inlet 32 and the waste-fluid outlet 34. Therefore, the pump 24 acts on the flow paths 20, 22 at a point which is more proximal to the wound dressing 14 than the treatment-fluid source 16 or the waste container 18 is. In other words, the pump 24 is interposed along the flow paths 20, 22, between the wound dressing 14 and the treatment-fluid inlet 26 and/or the waste-fluid outlet 34. The pump is or acts fluidly downstream of the treatment-fluid source 16 and upstream of the wound dressing 14 and/or fluidly downstream of the wound dressing 14 and fluidly upstream of the waste-container 18.

In this instance, the peristaltic pump 24 comprises a single pump head 44 with multiple pump rollers 45. The pump 24 further comprises a motor 46 with a drive shaft 47 which rotates the pump head 44. Each pump roller 45 is positioned to compress the tubing 30 so tubing walls interengage or contact each other and fonn a seal. Between two seals formed by two rollers 45, a known volume of fluid is defined. The two rollers 45 rotate around thehead, moving the seals and thus the volume of fluid along the tubing 30 and/or flow path 20, 22 so as to move the volume of fluid in the direction of pumping. Therefore, the tubing 30 is wrapped around the pump head 44 so as to be engageable and operable by the pump rollers 45. The pump head 44 and rollers 45 may be attached to the tubing 30 and may be changed when the device 12 is changed, although alternatively the pump head 44 is separate to the tubing 30.

The pumping means 24 includes a controller to control the treatment-fluid-application condition, and a monitoring means, monitoring element or monitor for monitoring a volume of treatment fluid pumped to the treatment-fluid outlet 28 during the treatment-fluid-application condition. Preferably, the pump of the pumping means 24 has a pumping cycle during which a known or determinable volume of fluid is pumped, for example the volume, or a multiple thereof; of fluid between two rollers 45. The number of pumping cycles or pump revolutions carried out by the pumping means 24 is determinable by the monitoring means, for example by an electronic counting means or sensor, to determine the volume of treatment fluid pumped to the treatment-fluid outlet 28. Whilst a pumping cycle is described, it will be appreciated that the pump may have a fluid flow rate or pumping rate. The monitoring means may detennine or time the duration the pump has been operating at said fluid flow rate so as to determine the volume of fluid pumped. In this instance the monitoring means would include a timer.

The monitoring means or monitor may comprise a physical or hardware component, or a user observing the pumping means, measuring the number of pumping cycles and/or calculating the volume. Preferably, however, the monitoring means comprises an algorithm, software or a computer program which is able to automatically count or track the number of pumping cycles and determine the volume and/or the flow rate of treatment fluid delivered to the wound, based on a diameter of the tubing 30. The controller is preferably linked to the monitoring means, the treatment-fluid-application condition being terminable by the controller when the monitoring means determines a predetermined volume of treatment fluid has been pumped. This may be an automatic process and may be achieved by the controller switching the pump off or reversing the pumping direction of the pump. The controller and monitoring means may preferably be electronic. The monitor may not in fact directly monitor the pump, instead monitoring the controller and the instructions passed from the controller to the pump.

The controller may include a user interface, for example to allow for a user to set a desired volume of treatment fluid to be applied to the wound dressing 14 and/or to stop the pumping means 24 from operating. The monitoring means may include a display or indicator to indicate to a user the volume of treatment pumped via the pumping means 24.

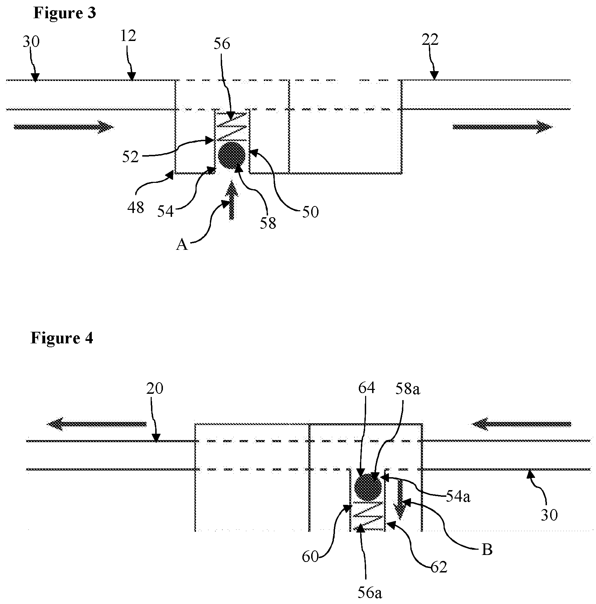

Referring in particular to Figure 3, the device 12 preferably further comprises a negative pressure regulator 48 in the waste-fluid flow path 22 and/or the treatment-fluid flow path 20. The negative pressure regulator 48 comprises a negative pressure relief valve 50 configured to be automatically openable to the exterior of the flow path 22 to increase pressure at the wound dressing 14 and automatically closeable to maintain pressure. Here the negative pressure relief valve 50 is openable to the exterior of the tubing 30. The negative pressure relief valve 50 preferably comprises a valve body 52 or side tube which provides a flow path to the exterior of the tubing 30 via an opening 54. In the side tube is a biasing element 56, which is here a spring such as a coil spring, and a sealing element 58 which may be deformable and/or compressible.

The spring 56 biases the sealing element 58 towards the opening 54 and away from the tubing 30. When the vacuum in the tubing 30 exceeds a predetermined level, the sealing element 58 is drawn towards the tubing 30 and away from the opening 54. This compresses the spring 56. This therefore creates an air or fluid flow path, the direction of which is illustrated by arrow A, between the exterior and interior of the tubing 30 via the opening 54. Airis therefore drawn into the tubing 30 which increases the pressure. Once the pressure has increased so that the vacuum is less than the predetermined level, the spring 56 forces the sealing element 58 towards the opening 54 and closes the opening 54. Therefore, pressure is maintained at or close to the predetermined level. An equilibrium may be reached whereby the spacing of the sealing element 58 and opening 54 allows the same flow rate of air in as is being drawn out by the pump. Here the sealing element 58 in the side tube is proximal to the tubing 30 as compared to the opening 54 and the biasing element 56 biases the sealing element 58 between the tubing 30 and the opening 54. However, the biasing element 56 may be attached or attachable between the sealing element 58 and the opening 54. Treatment fluid flowing through the tubing 30 during the treatment-fluid application condition or the waste-fluid removal condition forces the sealing element 58 against the opening 54 to prevent loss of fluid.

Referring to Figure 4, the device 12 may further comprise a positive pressure regulator 60, liquid pressure regulator or treatment-fluid pressure regulator, in the treatment-fluid flow path 20 and/or the waste-fluid flow path 22, the positive pressure regulator 60 regulates treatment fluid pressure at the treatment-fluid outlet 28 during the treatment-fluid-application condition. The positive pressure regulator 60 includes a vent conduit 62 having a positive pressure relief valve 64, the vent conduit 62 being fluidly communicable with the waste container 18 and/or the treatment-fluid source 16 to vent excess treatment fluid. The positive pressure relief valve 64 is similar to the negative pressure relief valve 50. However, the positive pressure relief valve 64 is configured so that a sealing element 58a is proximal to the tubing 30 as compared to or relative to a biasing element 56a or spring. When the pressure in the tubing 30 exceeds a predetermined level, the sealing element 58a is forced away from an opening 54a, compressing the spring 56a and opening a fluid or liquid pathway, the direction of which is illustrated by arrow B, between the tubing 30 and the vent conduit 62 via the opening 54a. Excess treatment fluid flows via the vent conduit 62 to the waste container 18, back to the treatment-fluid source 16 or to another waste container 18. This decreases fluid treatment pressure within the tubing 30 to the predetermined level. The spring 56a then biases the sealing element 58atowards the opening 54a to seal the opening 54a and prevent or limit reduction of fluid pressure below the predetermined level.

In the present embodiment the negative pressure regulator 48 and the positive pressure regulator 60 are positioned at or adjacent to each other and may have a single housing. However, alternatively that they may be spaced apart. Additionally, it will be appreciated that either or both pressure regulators may not be necessary for the device to function.

The wound dressing 14 preferably comprises a wound filler 66 for contacting a wound surface. The wound filler 66 may comprise a hydrophobic reticulated polyurethane foam. One purpose of the wound filler 66 is to facilitate the distribution of the treatment fluid being instilled into the wound as well as the distribution of pressure in the negative-pressure condition. Other wound filler 66 materials could include hydrophilic materials such as gauze or other porous materials. In this instance the materials will have a wicking effect through capillary action in the treatment-fluid-application condition, waste-fluid-removal and negative-pressure condition. Other materials may include Polyvinyl Alcohol PVA foam which is open cell and has a high absorbency. Bioresorbable, biodegradable or natural dissolving materials may also be used as a wound filler 66, these materials can be left in the wound and can act as a scaffold for new cell growth, examples of these materials include polylactic acid PLA, polyglycolic acid PGA, collagen, alginate, soy protein. In cases where it is important to maintain a consistent moisture within the wound the wound filler 66 may be in the fonn of a hydrogel or hydrocolloid.

In the case of foam based wound fillers 66 a coating may be applied to the foam at the manufacturing stage to provide antimicrobial properties or to further enhance wound cell generation coatings can include silver, zinc and magnesium. Additional passive coatings may be applied to the wound filler 66 that are activated by an agent in the instil fluid.

A non-adherent interface layer such as Mepitel RTM by Mölnlycke RTM Health Care may be used to prevent the foam sticking to the wound when it is removed.

A flexible film layer 68 or cover provides a seal and a barrier between the wound site and the external environment. This film layer 68 is placed over the wound filler 66 and adhered to surrounding intact skin of a patient. The film layer 68 may have adhesive over its entire surface or it will have a selective adhesive border or it may be secured to the intact skin by another means such as a hydrogel, hydrocolloid or silicon gasket.

The film layer 68 is preferably impermeable to liquid but allows moisture vapour transmission at a typical rate of 240 to 500 grams per metre squared per 24 hours. The film layer 68 preferably includes a film opening therethrough to allow connection of the tubing 30. The film sealed to the skin may therefore define a wound dressing chamber therebetween.

The device 12 preferably further comprises a wound-dressing connector 70 for connecting the wound dressing 14 to the treatment-fluid outlet 28 and/or the waste-fluid inlet 32. The wound-dressing connector 70 is a tube connector and is sealingly engageable with the opening which defines the treatment-fluid outlet 28 and the waste-fluid inlet 32, and sealingly engageable with the film opening. The wound-dressing connector 70 provides a means of connecting the tubing 30, and thus the treatment-fluid flow path 20 and the waste-fluid flow path 22, to the wound dressing 14 to maintain a seal and allow waste fluid or air to be withdrawn from the dressing and treatment fluid instilled into the dressing. The wound-dressing connector 70 may be attached to the film layer 68, for example the connector 70 may be adhered to the film layer 68 either from the top or underneath. Alternatively, the wound-dressing connector 70 may be integrally formed with the film layer 68, for example being co-moulded, welded or bonded to the film layer 68 as part of the manufacturing process. It can be manufactured from a range of bio-compatible polymers including PVC, TPU and silicone. The wound-dressing connector 70 may extend into the wound dressing 14, for example into the wound filler 66, to allow treatment fluids to penetrate to the base of the wound and/or wound dressing 14.

The tubing 30 connects to and may be received by the wound-dressing connector 70. The tubing 30 may be bonded into the connector at the manufacturing stage but alternatively may be connected in situ by means of a push fit, barbed, Luer lock connector or similar.

The treatment-fluid source 16 preferably comprises a treatment container which contains treatment-fluid. The treatment container may include flexible walls, and therefore may be a bag, although alternatively the treatment container may have rigid wall. The treatment container is preferably sealed and/or air tight, with the exception of an opening or port to connect to the treatment-fluid inlet 26, although in other instances it may be open. The treatment container may also have a valve or outlet, to vent air, if desired. The waste container 18 may be similarly formed as the treatment fluid container.

In this arrangement, the treatment-fluid source 16 and/or treatment container is separably connectable, engageable or fastenable to the treatment-fluid inlet 26 and/or the tubing 30. This allows an empty treatment container to be easily replaceable, for instance with a full treatment container. Preferably, the treatment-fluid source 16 and/or treatment container is connectable via a connector-element for facilitating the engagement of the treatment-fluid source 16 and/or treatment container with the treatment-fluid inlet 26 and/or the tubing 30. The connector-element preferably comprises at least one connector-valve. Said at least one connector-valve may be at least one of a pinch valve, solenoid or rotary valve, actuatable stopper, bung, a check or one-way valve such as a ball check valve, diaphragm check valve, swing check valve, clapper valve, a stop-check valve, lift-check valve, in-line check valve, duckbill valve, pneumatic non-return valve or any other mechanism suitable to prevent or inhibit treatment fluid flow through the connector-element when the treatment-fluid source 16 and/or treatment container is disengaged from the treatment-fluid inlet 26 and/or the tubing 30. The connector-element and/or the at least one connector-valve may be associated with at least one of the treatment-fluid inlet 26, the tubing 30, the treatment-fluid source 16 and the treatment container.

The waste container 18 is similar to the treatment-fluid source 16, by being separably connectable via a further connector-element, comprising at least one connector-valve, to easily replace a full waste container 18 with an empty waste container 18. Detailed description of the further connector-element and the connector-valve is omitted for brevity. Unlike the treatment-fluid source 16, the waste container 18 is separably connectable or fastenable to the waste-fluid outlet 34 and/or the tubing 30, rather than the treatment-fluid inlet 26.

The treatment fluid may otherwise be referred to as instillation fluid, instillate or instillation and may include saline solution, cleansing fluid or an active wound treatment substance. Whilst fluid is described, it will be appreciated that the treatment fluid may include solid matter, for example in suspension, such as cells and/or tissue. Additionally, the device 12 is preferably for use with liquid treatment, rather than gaseous treatment, although gaseous treatment may also be considered. Waste fluid may include treatment fluid which has been applied to the wound dressing 14 or wound exudate.

In use, the wound-dressing conditioning device 12 is fluidly connected to or fluidly communicated with a wound dressing 14, a treatment-fluid source 16 and a waste container 18. The wound dressing 14 is then attached to the patient, over and/or in the wound of the patient to be treated.

As shown particularly in Figure 1, the selector valve 42 is then actuated to fluidly connect the dual tubing-section 36 with the treatment tubing-section 38, the treatment-fluid inlet 26 and the treatment-fluid source 16. The pumping means 24 is then activated to pump treatment fluid from the treatment-fluid source 16, along the treatment-fluid flow path 20 and to the wound dressing 14. This is the treatment-fluid application condition. It will be appreciated that the tubing 30 and/or wound dressing 14 may be required to be evacuated or partially evacuated of air before pumping treatment fluid therethrough. This evacuation may be achieved using the negative-pressure condition, and this may be better understood hereinafter. Alternatively, before sealing the wound dressing 14 to the skin of the patient, the pumping means 24 may be operated to fill at least part of the tubing 30 with treatment fluid. However, it will also be appreciated that neither of these precautions may be taken and the air in the tubing 30 may be compressed, dissolved in the treatment fluid and/or resulting air bubbles may not reduce the effectiveness of the treatment.

The pumping means 24 is operated via the controller and the user may input into the controller a predetermined volume of treatment fluid to be pumped to the wound dressing 14. The pumping means 24 then pumps the treatment fluid and once the monitoring means determines that the predetermined volume of treatment has been pumped, the pump may then be deactivated. The pumping means 24 therefore directly pumps the treatment fluid. Should excessive treatment fluid be applied so that fluid pressure within the device 12 exceeds a predetermined level, the positive pressure regulator 60 allows the venting of excess treatment fluid in the way previously described.

With the treatment fluid in the wound dressing 14, a dwell time may then be provided. As shown particularly in Figure 2, after the dwell time, the selector valve 42 is actuated to fluidly connect or communicate the dual tubing-section 36 and the waste tubing 30 section. The pump is then operated, for example by the controller, to pump waste fluid from the wound dressing 14 to the waste container 18 along the waste-fluid flow path 22. This is the waste-fluid-removal condition. Such pumping may be monitored, for example to measure exudation rate of the wound or to verify when all waste-fluid has been removed from the wound.

After removing all or substantially all waste fluids from the wound dressing 14, the pumping means 24 may then continue to operate to pump 24 to apply a negative pressure or a vacuum to the wound dressing 14 and/or the wound. Therefore, the wound dressing 14 chamber is evacuated of air and any subsequent waste fluids exuded by the wound. This is the negative-pressure condition. The negative pressure may be regulated by the negative pressure regulator 48. The air is preferably evacuated to the waste container 18, although it will be appreciated that it may in fact be evacuated to the treatment-fluid container 16.

Although not shown, to remove large quantities of air, the device may require a further, more powerful pumping means. For example, a conventional air pump may be fluidly connected to the wound dressing 14 or the waste-fluid flow path 22.

The negative pressure and/or vacuum may then be maintained at the wound and/or wound dressing 14 for a predetennined period of time. The pump 24 may be deactivated once the desired negative pressure at the wound or wound dressing is reached and periodically reactivated by the controller to ensure the low pressure is maintained to account for any leakage.

After maintaining negative pressure for the predetermined period of time, the wound-conditioning apparatus 10 may be removed. Altematively, the treatment-fluid application condition may be reactivated, followed by the waste-fluid-removal condition and then the negative pressure condition. This cycle may be repeated as required and the cycling may be automatic, for example the controller using the monitoring means to determine the volume of treatment fluid pumped and a timer to time the duration of the negative pressure condition. Alternatively, the negative pressure condition may not be activated and the user may only use the device 12 to apply treatment fluid to the wound and then remove substantial waste fluids, switching off the pump 24 or applying further treatment fluid once substantially all waste fluids have been removed.

Whilst described as being defined by the same extent of tubing 30, it will be appreciated the treatment-fluid flow path and waste-fluid flow path may comprise separate lumina within the same tubing. The tubing, for example the dual tubing-section may be extruded in a multi-lumen format with both the treatment-fluid flow path and the waste-fluid flow path in the same profile but hermetically separated. Additional lumina may be included for extra flow paths or for pressure sensing means.

Although in this arrangement, the treatment and waste containers 18 are temporarily engageable with the treatment-fluid inlet 26, the tubing 30 and/or the waste-fluid outlet 34, it could be envisaged that at least one or both container may not be disengageable from the treatment-fluid inlet, tubing and/or waste-fluid outlet.

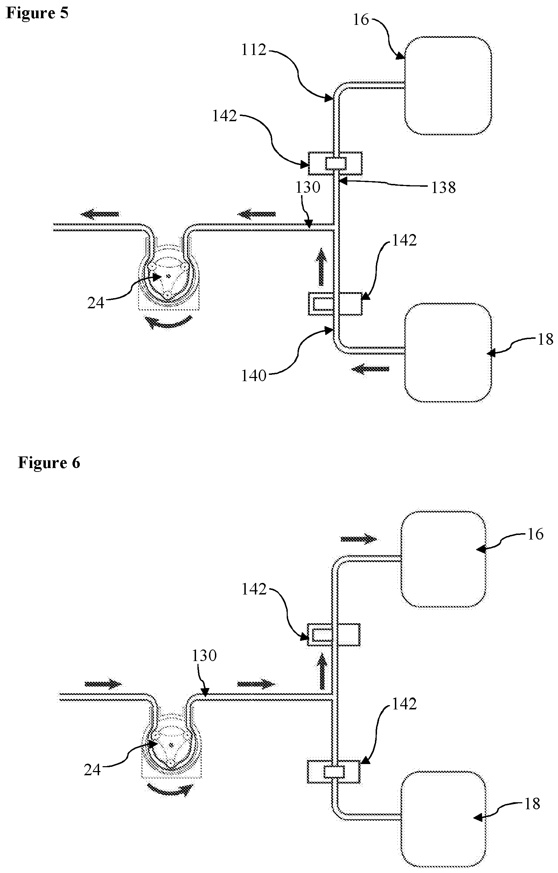

Referring now to Figures 5 and 6, there is shown a second embodiment of the wound-dressing condition device 112 in which the selector valve 142 comprises a valve 142 in each of the waste tubing-section 140 and the treatment tubing-section 138. Elements which are similar or identical to those of the preceding wound-dressing conditioning device 12 are denoted by the same or similar reference number with 100 added where necessary, and further detailed description is omitted.

The valves 142 are preferably pinch valves, and therefore operate by compressing or constricting the tubing 130 so that the walls interengage and close a bore of the tubing 130. Alternatively diaphragm valves, ball valves or any other type of valve may be considered. When one valve is open, preferably the other valve is closed.

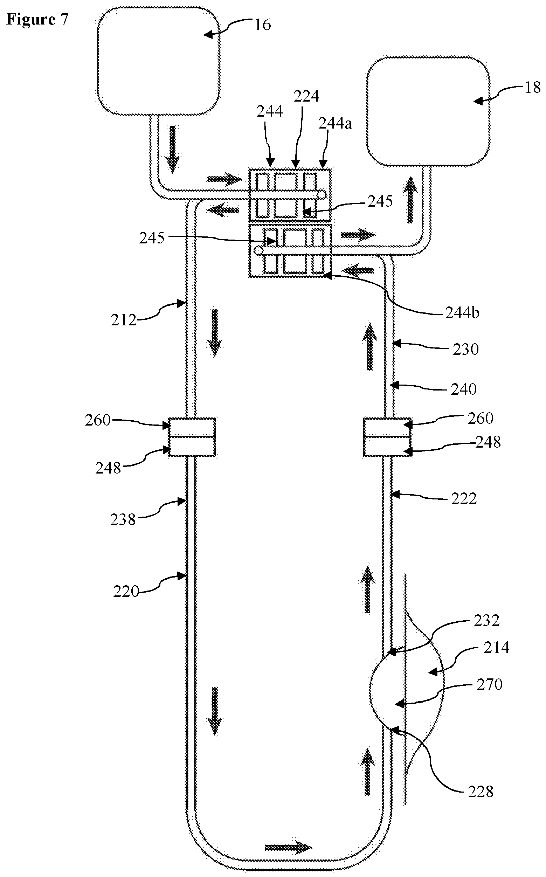

Referring to Figure 7, there is shown a third embodiment of the wound-dressing conditioning device 212 wherein there is no dual tubing-section and the treatment-fluid flow path 220 is entirely, or substantially entirely, defined by a treatment tubing-section 238 and the waste-fluid flow path 222 is entirely, or substantially entirely, defined by a waste tubing-section 240. Elements which are similar or identical to those of the initial wound-dressing conditioning device 12 are denoted by the same or similar reference number with 200 added where necessary, and further detailed description is omitted.

The treatment-fluid outlet 228 and the waste-fluid inlet 232 of the device 212 are also separate and distinct. The wound-dressing connector 270 may be adapted to connect the treatment-fluid outlet 228 and the waste-fluid inlet 232 to the wound dressing 214 in a variety of configurations such as side by side, opposing each other or spaced apart. In the present embodiment the treatment-fluid outlet 228 and the waste-fluid inlet 232 are communicated with the wound dressing 214 in an opposing configuration.

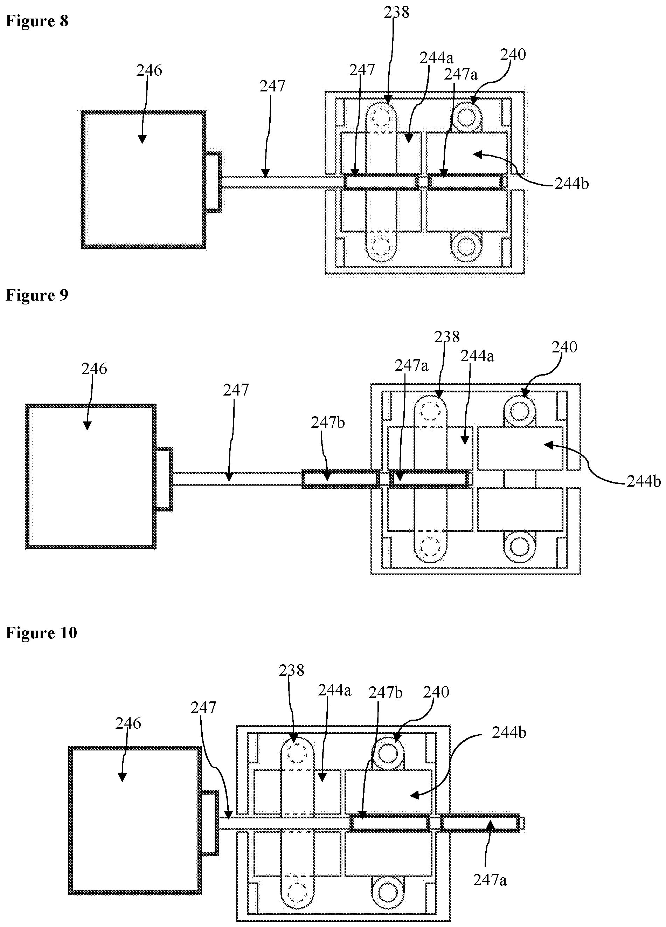

Referring to Figures 8 to 10, the pumping means 224 is a pump which has at least two pump heads 244, the pump 224 preferably being a peristaltic pump. Here the pump 224 has a treatment pump-head 244a and a waste pump-head 244b. The treatment pump-head 244a and the waste pump-head 244b are selectably operable by the drive shaft 247 of the motor 246 of the pump. Therefore, the treatment pump-head 244a and the waste pump-head 244b may be operated by the drive shaft 247 at the same time, as shown in Figure 8. Alternatively, the treatment pump-head 244a can be operated by the drive shaft 247 and the waste pump-head 244b not operated, as shown in Figure 9. Furthermore, the waste pump-head 244b can be operated by the drive shaft 247 and the treatment pump-head 244a not operated, as shown in Figure 10.

This selective operation is enabled by the drive shaft 247 being axially moveable and the treatment pump-head 244a and the waste pump-head 244b being axially off-set from one another. The drive shaft 247 has two drivers, a distal driver 247a and a proximal driver 247b. The treatment pump-head 244a is proximal to the motor 246 as compared to the waste pump-head 244b. Therefore, to drive both pump heads 244a, 244b, the distal driver 247a is received in the waste pump-head 244b and the proximal driver 247b is received in the treatment pump-head 244a. To drive only the treatment pump-head 244a, the distal driver 247a is received in the treatment pump-head 244a and the proximal driver 247b is proximal to the motor 246 as compared to both pump heads 244. To drive only the waste pump-head 244b, the proximal driver 247b is received in the waste pump-head 244b and the distal driver 247a is distal to the motor 246 as compared to both pump heads 244a, 244b.

The motor 246 of the pump need not to be reversible and both pump heads 244 rotate in the same direction. The treatment tubing-section 238 is arranged around the treatment pump-head 244a so as to pump the treatment from the treatment-fluid inlet 226 to the treatment-fluid outlet 228. The waste tubing-section 240 is arranged around the waste pump-head 244b to pump treatment from the waste-fluid inlet 232 to the waste-fluid outlet 234. In this way the treatment tubing-section 238 is arranged around the treatment pump-head 244a in an opposing direction as compared to the arrangement of the waste tubing-section 240 around the waste pump-head 244b. This is shown in Figure 7 where, from the treatment-fluid inlet 226, the treatment tubing-section 238 extends over the treatment pump-head 244a, wraps around the pump head 244 and leaves the treatment pump-head 244a from underneath. In contrast, from the waste-fluid inlet 232, the waste tubing-section 240 extends under the waste pump-head 244b, wraps around and leaves the waste pump-head 244b from above. However, it will be appreciated that this arrangement may be reversed.

Preferably both the treatment-fluid flow path 220 and the waste-fluid flow path 222 have one negative pressure regulator 248 and one positive pressure regulator 260. However, the treatment-fluid flow path may only have a positive pressure regulator and waste-fluid flow path may only have a negative pressure regulator.

The third embodiment of the device 212 is used in a similar way as the first embodiment 12. This is with the exception that the treatment-fluid application condition and the waste-fluid removal condition can be operated at the same time by operating both pump heads 244a, 244b. This allows for treatment fluid to be pumped to the wound dressing 214 along the treatment-fluid flow path 220 before being removed, after application to the wound, as a waste fluid along the waste-fluid flow path 222. This allows for efficient flushing of the wound.

Alternatively, the treatment pump-head 244a may be connected to the motor 246 and the waste pump-head 244b disconnected, resulting in treatment fluid being applied to the wound dressing 214. In the instance that the pump head 244 is a peristaltic pump head, a seal is maintained in the waste-fluid flow path 222 by the rollers 245 of the waste pump-head 244b. However, if a peristaltic pump head 244 is not used, a separate valve may be used to seal the waste-fluid flow path. The applied treatment can then be left to dwell before connecting the waste pump-head 244b to the drive shaft 247 to remove waste fluids.

The amount of treatment-fluid applied may be monitored or metered in the same way as previously described.

The negative pressure or vacuum condition requires for all waste fluids to first be removed. Therefore, the supply of treatment fluid to the wound dressing 214 will be required to be stopped. This may preferably be enabled by disconnecting the treatment pump-head 244a from the drive shaft 247. In the instance that the pump head 244 is a peristaltic pump head, a seal is maintained in the treatment-fluid flow path 220 by the rollers 245 of the treatment pump-head 244a. However, if a peristaltic pump head is not used, as separate valve may be used to seal the treatment-fluid flow path. This allows for the tubing 230 up to the treatment pump-head 244a and the wound dressing 124 chamber to be evacuated. Therefore, the negative-pressure condition can be applied to the wound.

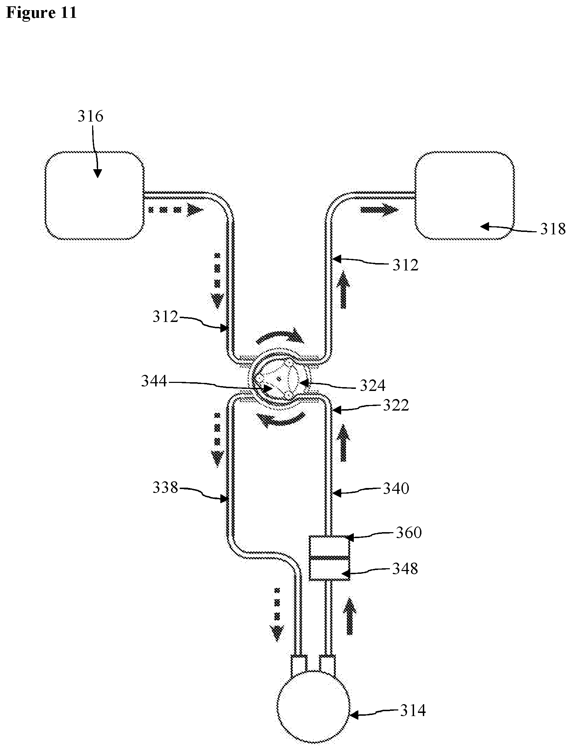

Referring now to Figure 11, there is shown a fourth embodiment of the wound-dressing conditioning device 312 in which the pumping means 324 is a pump with a single pump head 344. Elements which are similar or identical to those of the preceding wound-dressing conditioning device 312 are denoted by the same or similar reference number with 100 added where necessary, and further detailed description is omitted.

The treatment tubing-section 338 and the waste tubing-section 340 are preferably arranged around the single pump head 344 in opposing configurations to allow for opposing pumping directions on each tubing section 338, 340. Here the waste-fluid flow path 22 has the negative pressure regulator 348 and the positive pressure regulator 360, although the treatment fluid-flow path may additionally or alternatively have the negative pressure regulator and the positive pressure regulator.

The fourth embodiment 312 may be used similarly to the preceding embodiment 212. However, the treatment-fluid application condition is operated concurrently with the waste-fluid removal condition, due to the inability to isolate pumping on either flow path. To apply a negative pressure condition or vacuum condition to the wound dressing 314, the treatment-fluid container 316 may be required to be emptied of treatment fluid. The treatment-fluid container 316 would then also have a negative pressure applied thereto. Alternatively, a valve may be positioned on the treatment tubing-section to prevent the treatment fluid container from being evacuated. This valve may be positioned between the pump and the treatment-fluid container.

It will be appreciated that dual lumen variants of the first embodiment of the device 12 may function in a similar or identical way to the fourth embodiment of the device 312.

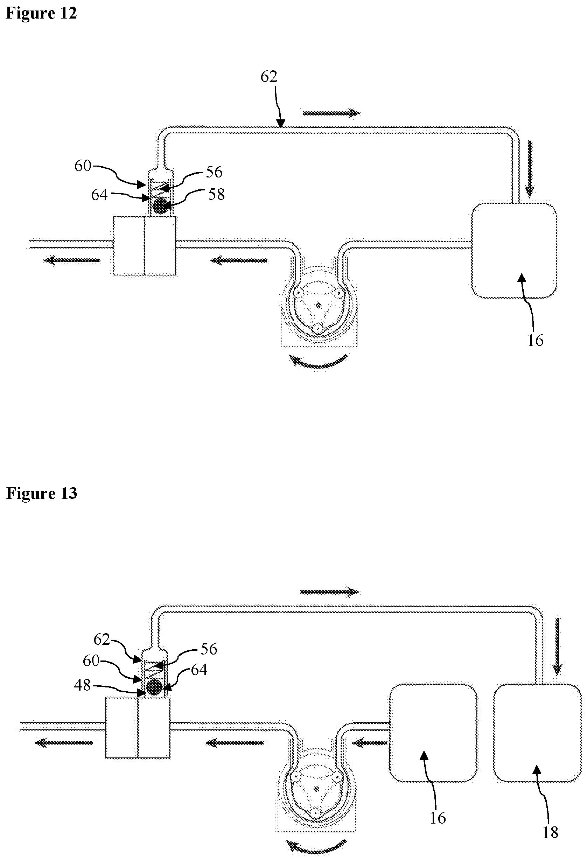

Referring to Figure 12, there is shown a further configuration of the positive pressure regulator 60, illustrating the vent conduit 62 and the positive pressure relief valve 64 comprising the biasing element 56 and the sealing element 58. The vent conduit 62 is fluidly communicated directly with the treatment-fluid source 16.

Referring to Figure 13, there is shown a further configuration of the positive pressure regulator 60, illustrating the vent conduit 62 and the positive pressure relief valve 64 comprising the biasing element 56 and the sealing element 58. The vent conduit 62 is fluidly communicated directly with the waste container 18.

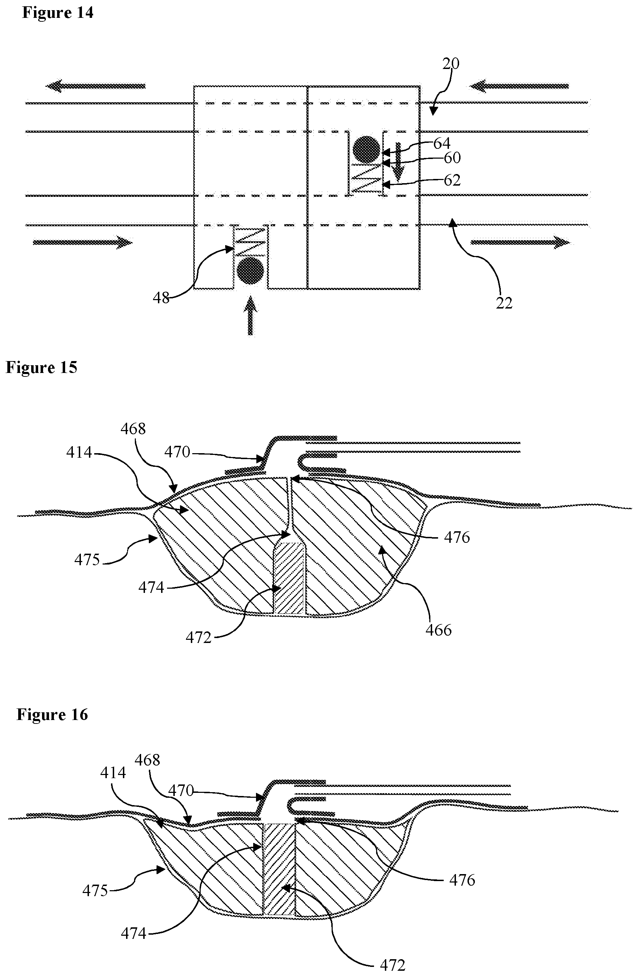

Referring to Figure 14, there is shown a further configuration of the positive pressure regulator 60 and the negative pressure regulator 48. As illustrated, the vent conduit 62 of the positive pressure relief valve 64 is fluidly communicated with the waste fluid flow path. This allows for excess treatment-fluid to be directed towards the waste container 18. Such an embodiment could not be used with a dual tubing-section 36, unless the dual tubing-section 36 comprised a dual lumen.

It will be appreciated that the device may comprise a pressure sensor at the wound dressing to ensure that the wound dressing is at the correct negative pressure or vacuum. Such a sensor may be electronic and may be required to be connected to the wound dressing or the waste-fluid flow path, for example via tubing or a separate lumen.

Alternatively, referring to Figures 15 and 16, the pressure sensor may comprise a visual indicator 472 positioned in the wound dressing 414, for example in the wound filler 466, which may be foam. In use the wound filler 466 is positioned at or in the wound 475. When the wound dressing 414 is under a predetermined amount of negative pressure, the wound filler 466 is compressed which reveals the visual indicator 472. The visual indicator 472 is visible through the transparent or translucent film 468 or covering of the wound dressing 414. The visual indicator 472 should be less compressible or defonnable than the wound filler 466. If the wound dressing 414 is under insufficient negative pressure, the wound filler 466 is not compressed sufficiently and the visual indicator 472 is at least in part obscured. This provides a means of visually verifying whether the wound dressing 414 is under sufficient negative pressure. The visual indicator 472 preferably has a three-dimensional shape, and therefore is not flat. As such the visual indicator 472 extends through at least part of a depth of the wound filler 466, for example extending through at least a majority of the depth of the wound filler 466. Here, the visual indicator 472 is cylindrical or substantially cylindrical, although hollow cylinders, rectangular or triangular prisms may also be considered. However, it will be appreciated that the visual indicator 472 may be flat, for example being a disc, or a ring, although a square, a rectangle, any other polygon, and/or at least partly curved shape may also be envisaged. The visual indicator 472 may be coloured, for example blue or red, and preferably has a different colour to the wound filler 466, although it may alternatively be a light emitting element. The visual indicator 472 may even change colour or display an indicator upon detecting a determined pressure.

In this instance, the wound filler 466 may have at least one aperture 474 extending to the visual indicator 472 from the exterior of the wound filling. The aperture 474 extends substantially linearly and substantially perpendicularly to the visual indicator 472 and/or the film 468 such that a user or medical staff may easily observe the visual indicator 472 through the aperture 474 when the wound filler 466 is under negative pressure. The aperture 474 may be between 10 mm and 30 mm deep or in diameter. The aperture 474 may be circular or disc-shaped in cross-section, although other shapes, circular or non-circular may be possible. Additionally, the aperture may have anon-uniform cross-section, for example tapering towards the wound or the film 468.

The wound filler 466 is preferably contractible by application of pressure given that the wound filler 466 is preferably an aerated material or a foam. In this way, the wound filler 466 occupies a smaller volume when under lower pressure than when under higher pressure. The aperture 474, or an opening 476 thereof, may grow in diameter when negative pressure is applied via contraction of the wound filler 466, thereby allowing the visual indicator 472 to be more easily visible. The aperture 474 may be shrunk or closed such that the visual indicator 472 is entirely or substantially entirely obscured when positive pressure is applied via expansion of the wound filler. When the wound filler 466 is at atmospheric pressure, it will be appreciated that the aperture 274 may be closed or substantially closed so that the visual indicator 472 is occluded from view. A ratio of the respective diameters of the visual indicator 472 and the aperture 474, and/or the area of the visual indicator 472 visible may be used to determine the pressure applied to the wound.

However, it will be appreciated that an aperture may not be required. For example, the visual indicator may be positioned on a surface of the wound filler. When positive pressure is applied, the wound filler may expand so as to at least in part envelop the indicator.

The visual indicator 472 may be positioned under the wound dressing connector 470, which is preferably transparent. Alternatively, the visual indicator 472 can be positioned adjacent to the wound dressing connector 470 as dictated by the wound filler 466. The position of the aperture 474 in the wound filler 466 relative to edges of the wound filler, and therefore the edges of the in use wound, can be adjusted by trimming the wound filler 466 to off-set the aperture 474 from the centre of the wound, wound filler 466 or the position of the wound dressing connector 470. Alternatively, the wound filler 466 may be supplied with the aperture 474 in a pre-detennined position at the manufacturing stage, for example off set from a centre of the wound filler 466.

The visual indicator 472 could be manufactured from the same polymeric material as the wound filler 466 but in a different colour, typically the wound filler 466 is made from a reticulated polyurethane foam preferably with a pore size in the region of 40 to 45 pores per inch (ppi). If the visual indicator 472 and wound filler 466 are made from the same material then the opening 476 of the aperture 474 is enlarged when under negative pressure which reveals the visual indicator, even if the visual indicator 472 is contracted by the same amount as the wound filler 466.

The visual indicator 472 could also be manufactured in a polymeric material with substantially different properties than the wound filler 466 to accentuate dimensional differences when subjected to a negative pressure to improve visibility of the status of the indicator. The properties of the visual indicator 472 could be varied by using a different polymeric foam such as a Polyvinyl Alcohol, this has the advantage of being substantially rigid until it becomes moist. Alternatively, the properties can be varied by using a polyurethane foam with a different porosity than the wound filler 466. The pores per inch of the visual indicator 472 could be varied between 10 ppi and 60 ppi, and other physical properties that could be varied include density and hardness.

Additionally, the device and/or the monitoring means may comprise a flow-rate sensor to monitor a flow rate of treatment fluid. The flow-rate sensor may therefore be positionable in the treatment-fluid flow path, although it may be at a parallel flow path in a separate tube or lumen.

It is therefore possible to provide a wound-dressing conditioning device able to apply a positive pressure, for example to apply treatment fluid, and a negative pressure, for example to remove waste fluid and/or apply a vacuum. The treatment fluid is pumped directly by a pump and therefore the volume of treatment pumped is measurable and monitorable by the device.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined by the appended set of claims.

## Claims

1. A wound-dressing conditioning device (312) for conditioning a wound dressing (314), the device comprising:
a treatment-fluid flow path having a treatment-fluid inlet for fluidly communicating with a treatment-fluid source (316), and a treatment-fluid outlet for fluidly communicating with the wound dressing (314);
a waste-fluid flow path (322) having a waste-fluid inlet for fluidly communicating with a wound dressing (314), and a waste-fluid outlet for fluidly communicating with a waste-fluid container (318); and
a pumping means (324) at or adjacent to the treatment-fluid flow path and the waste-fluid flow path (322), the in-use pumping means (324) having a treatment-fluid-application condition for pumping treatment fluid from the treatment-fluid inlet to the treatment-fluid outlet, a waste-fluid-removal condition for pumping waste fluid from the waste-fluid inlet to the waste-fluid outlet, and a negative-piessure condition for applying negative pressure to the wound dressing (314);
a peristaltic pump (324) having a controller to control the treatment-fluid-application condition, and a monitoring means for monitoring a volume of treatment fluid pumped to the treatment-fluid outlet during the treatment-fluid-application condition,
wherein the waste-fluid flow path is entirely or substantially entirely defined by a waste tubing-section (340) and the treatment-fluid flow path is entirely or substantially entirely defined by a treatment tubing-section (338), the waste tubing-section (340) and the treatment tubing-section (338) being separate,
**characterised in that** the peristaltic pump (324) has one pump head (344), the treatment tubing section (238) and the waste tubing section being positioned in opposing orientations on the pump head (344).

2. A wound-dressing conditioning device (312) as claimed in claim 1, wherein the peristaltic pump (324) has a pumping cycle during which a known volume of fluid is pumped, and the number of pumping cycles carried out by the pumping means (324) is determinable by the monitoring means to determine the volume of treatment fluid pumped to the treatment-fluid outlet.

3. A wound-dressing conditioning device (312) as claimed in claim 1 or claim 2, wherein the controller is linked to the monitoring means, the treatment-fluid-application condition being terminable by the controller when the monitoring means determines a predetermined volume of treatment fluid has been pumped.

4. A wound-dressing conditioning device (312) as claimed in any one of the preceding claims, further comprising the wound dressing (314) connected to the treatment-fluid outlet and the waste-fluid inlet, wherein the wound dressing (314) includes a wound filler (466) and a visual indicator (472), the visual indicator having at least one visual difference to the wound filler (466) and being positioned at, in or on the wound filler (466), the wound filler (466) being contractible by negative pressure so as to at least in part expose the visual indicator (472) and/or expandable by positive pressure so as to at least in part cover the visual indicator (472).

5. A wound-dressing conditioning device (312) as claimed in claim 4, wherein the wound filler (466) comprises an aperture (474) which extends at least part-way therethrough, the indicator (472) being positioned in said aperture (474), and the wound filler (466) being contractible by negative pressure so as to enlarge an opening (476) of said aperture (474) and/or expandable by positive pressure so as to at least in part close an opening (476) of said aperture (474).

6. A wound-dressing conditioning device (312) as claimed in claim 4 or claim 5, wherein the wound filler (466) is more compressible and/or expandable than the visual indicator (472).

7. A wound-dressing conditioning device (312) as claimed in any one of the preceding claims, further comprising a negative pressure regulator (348) in the waste-fluid flow path (322) and/or the treatment-fluid flow path, the negative pressure regulator (348) comprising a valve configured to be automatically openable to the exterior of the flow path to increase pressure at the wound dressing (414), and automatically closeable to maintain pressure.

8. A wound-dressing conditioning device (312) as claimed in any one of the preceding claims, further comprising a positive pressure regulator (360) in the treatment-fluid flow path and/or the waste-fluid flow path (322), the positive pressure regulator (360) comprising a valve configured to be automatically openable to the exterior of the flow path to decrease pressure at the wound dressing (314), and automatically closeable to maintain pressure.

9. A wound-dressing conditioning device (312) as claimed in any one of the preceding claims, further comprising a valve on the treatment tubing-section configured to prevent passage of treatment fluid from the treatment-fluid inlet to the treatment-fluid outlet for allowing negative pressure to be applied to the wound dressing.

## Patentansprüche

1. Wundverbandkonditionierungsvorrichtung (312) zur Konditionierung eines Wundverbands (314), wobei die Vorrichtung umfasst:
einen Behandlungsfluidflussweg mit einem Behandlungsfluideinlass zur Fluidkommunikation mit einer Behandlungsfluidquelle (316) und einem Behandlungsfluidauslass zur Fluidkommunikation mit dem Wundverband (314);
einen Abfallproduktfluidflussweg (322) mit einem Abfallproduktfluideinlass zur Fluidkommunikation mit einem Wundverband (314) und einem Abfallproduktfluidauslass zur Fluidkommunikation mit dem Abfallproduktfluidbehälter (318); und
ein Pumpmittel (324) an dem oder angrenzend an den Behandlungsfluidflussweg und den Abfallproduktfluidflussweg (322), wobei das Pumpmittel (324) im Gebrauch einen Behandlungsfluidanwendungszustand zum Pumpen von Behandlungsfluid aus dem Behandlungsfluideinlass zu dem Behandlungsfluidauslass, einen Abfallproduktfluidentfernungszustand zum Pumpen von Abfallproduktfluid aus dem Abfallproduktfluideinlass zu dem Abfallproduktfluidauslass und einen Unterdruckzustand zum Anwenden eines Unterdrucks auf den Wundverband (314);
eine Peristaltikpumpe (324) mit einer Steuereinheit zum Steuern des Behandlungsfluidanwendungszustands und einem Überwachungsmittel zum Überwachen eines Volumens des Behandlungsfluids, das während des Behandlungsfluidanwendungszustands zu dem Behandlungsfluidauslass gepumpt wird,
wobei der Abfallproduktfluidflussweg vollständig oder im Wesentlichen vollständig durch einen Abfallproduktleitungsabschnitt (340) definiert ist und der Behandlungsfluidflussweg vollständig oder im Wesentlichen vollständig durch einen Behandlungsleitungsabschnitt (338) definiert ist, wobei der Abfallproduktleitungsabschnitt (340) und der Behandlungsleitungsabschnitt (338) getrennt sind,
**dadurch gekennzeichnet, dass** die Peristaltikpumpe (324) einen Pumpenkopf (344) aufweist, wobei der Behandlungsleitungsabschnitt (238) und der Abfallproduktleitungsabschnitt in entgegengesetzten Ausrichtungen an dem Pumpenkopf (344) positioniert sind.

2. Wundverbandkonditionierungsvorrichtung (312) nach Anspruch 1, wobei die Peristaltikpumpe (324) einen Pumpzyklus aufweist, während dem ein bekanntes Volumen des Fluids gepumpt wird, und die Anzahl von Pumpzyklen, die von dem Pumpmittel (324) durchgeführt werden, durch das Überwachungsmittel bestimmbar ist, um das Volumen des Behandlungsfluids, das zu dem Behandlungsfluidauslass gepumpt wird, zu bestimmen.

3. Wundverbandkonditionierungsvorrichtung (312) nach Anspruch 1 oder Anspruch 2, wobei die Steuereinheit mit dem Überwachungsmittel verbunden ist, wobei der Behandlungsfluidanwendungszustand durch die Steuereinheit aufhebbar ist, wenn das Überwachungsmittel bestimmt, dass ein vorbestimmtes Volumen des Behandlungsfluids gepumpt wurde.

4. Wundverbandkonditionierungsvorrichtung (312) nach einem der Ansprüche, ferner umfassend, dass der Wundverband (314) mit dem Behandlungsfluidauslass und dem Abfallproduktfluideinlass verbunden ist, wobei der Wundverband (314) einen Wundfüller (466) und einen optischen Indikator (472) einschließt, wobei der optische Indikator mindestens einen optischen Unterschied zu dem Wundfüller (466) aufweist und an, in oder auf dem Wundfüller (466) positioniert ist, wobei der Wundfüller (466) durch Unterdruck zusammenziehbar ist, um den optischen Indikator (472) zumindest zum Teil freizulegen, und/oder durch Überdruck ausdehnbar ist, um den optischen Indikator (472) zumindest zum Teil abzudecken.

5. Wundverbandkonditionierungsvorrichtung (312) nach Anspruch 4, wobei der Wundfüller (466) einen Durchtritt (474) umfasst, der sich zumindest zum Teil dort hindurch erstreckt, der Indikator (472) in dem Durchtritt (474) positioniert ist und der Wundfüller (466) durch Unterdruck zusammenziehbar ist, um eine Öffnung (476) des Durchtritts (474) zu vergrößern, und/oder durch Überdruck ausdehnbar ist, um eine Öffnung (476) des Durchtritts (474) zumindest zum Teil zu schließen.

6. Wundverbandkonditionierungsvorrichtung (312) nach Anspruch 4 oder Anspruch 5, wobei der Wundfüller (466) stärker zusammenziehbar und/oder ausdehnbar ist als der optische Indikator (472).

7. Wundverbandkonditionierungsvorrichtung (312) nach einem der vorhergehenden Ansprüche, ferner umfassend einen Unterdruckregler (348) in dem Abfallproduktfluidflussweg (322) und/oder dem Behandlungsfluidflussweg, wobei der Unterdruckregler (348) ein Ventil umfasst, das dazu konfiguriert ist, automatisch zu der Außenseite des Flusswegs öffenbar zu sein, um einen Druck an dem Wundverband (414) zu erhöhen, und automatisch schließbar ist, um den Druck aufrechtzuerhalten.

8. Wundverbandkonditionierungsvorrichtung (312) nach einem der vorhergehenden Ansprüche, ferner umfassend einen Überdruckregler (360) in dem Behandlungsfluidflussweg und/oder dem Abfallproduktfluidflussweg (322), wobei der Überdruckregler (360) ein Ventil umfasst, das dazu konfiguriert ist, automatisch zu der Außenseite des Flusswegs öffenbar zu sein, um einen Druck an dem Wundverband (314) zu senken, und automatisch schließbar ist, um den Druck aufrechtzuerhalten.

9. Wundverbandkonditionierungsvorrichtung (312) nach einem der Ansprüche, ferner umfassend ein Ventil an dem Behandlungsleitungsabschnitt, das dazu konfiguriert ist, einen Durchfluss des Behandlungsfluids aus dem Behandlungsfluideinlass zu dem Behandlungsfluidauslass zu verhindern, um zu ermöglichen, dass ein Unterdruck auf den Wundverband angewendet wird.

## Revendications

1. Dispositif de conditionnement de pansement (312) pour conditionner un pansement (314), le dispositif comprenant :
un trajet d'écoulement de fluide de traitement comprenant une entrée de fluide de traitement pour communiquer avec une source de fluide de traitement (316), et une sortie de fluide de traitement pour communiquer de manière fluidique avec le pansement (314) ;
un trajet inférieur de fluide usé (322) ayant une entrée de fluide usé pour une communication fluidique avec un pansement (314), et une sortie de fluide usé pour une communication fluidique avec un récipient de fluide usé (318) ; et
un moyen de pompage (324) situé au niveau ou à proximité du trajet d'écoulement du fluide de traitement et du trajet d'écoulement du fluide usé (322), ledit moyen de pompage (324) en utilisation, ayant une condition d'application de fluide de traitement pour pomper le fluide de traitement à partir de l'entrée du fluide de traitement vers la sortie de fluide de traitement, une condition d'élimination de fluide usé pour pomper le fluide usé à partir de l'entrée de fluide usé vers la sortie de fluide usé, et une condition de pression négative pour appliquer une pression négative au pansement (314) ;
une pompe péristaltique (324) comprenant un contrôleur pour contrôler la condition d'application du fluide de traitement, et un moyen de surveillance pour surveiller un volume de fluide de traitement pompé vers la sortie de fluide de traitement pendant la condition d'application du fluide de traitement,
dans lequel le trajet d'écoulement de fluide usé est entièrement ou sensiblement entièrement défini par une section de tubulure de fluide usé (340), et le trajet d'écoulement de fluide de traitement est entièrement ou sensiblement entièrement défini par une section de tubulure de fluide de traitement (338), lesdites section de tubulure de fluide usé (340) et section de tubulure de fluide de traitement (338) étant séparées ;
**caractérisé en ce que** la pompe péristaltique (324) comporte une tête de pompe (344), la tubulure de traitement (238) et la section de tubulure de fluide usé étant disposées selon des orientations opposées sur la tête de pompe (344).

2. Un dispositif de conditionnement de pansement (312) selon la revendication 1, dans lequel la pompe péristaltique (324) présente un cycle de pompage pendant lequel un volume connu de fluide est pompé, et le nombre de cycles de pompage effectués par le moyen de pompage (324) est déterminable par le dispositif de surveillance pour déterminer le volume de fluide de traitement pompé vers la sortie de fluide de traitement.

3. Un dispositif de conditionnement de pansement (312) selon la revendication 1 ou la revendication 2, dans lequel le contrôleur est relié aux moyens de surveillance, la condition d'application du fluide de traitement pouvant être interrompue par le contrôleur lorsque les moyens de surveillance déterminent qu'un volume prédéterminé de fluide de traitement a été pompé.

4. Un dispositif de conditionnement de pansement (312) selon l'une quelconque des revendications précédentes, comprenant en outre le pansement (314) relié à la sortie de fluide de traitement et à l'entrée de fluide usé, dans lequel le pansement (314) comprend un matériau de remplissage de plaie (466) et un indicateur visuel (472), ledit indicateur visuel présente au moins une différence visuelle par rapport au matériau de remplissage de plaie (466) et est disposé sur, dans ou à la surface dudit matériau de remplissage de plaie (466), le matériau de remplissage de plaie (466) étant contractable sous l'effet d'une pression négative de manière à au moins partiellement exposer l'indicateur visuel (472) et/ou expansible sous l'effet d'une pression positive de manière à au moins partiellement recouvrir l'indicateur visuel (472).

5. Un dispositif de conditionnement de pansement (312) selon la revendication 4, dans lequel le matériau de remplissage de plaie (466) comprend une ouverture (474) qui s'étend au moins partiellement à travers celui-ci, l'indicateur (472) étant disposé dans ladite ouverture (474), et le matériau de remplissage de plaie (466) étant contractable sous l'effet d'une pression négative de manière à agrandir une ouverture (476) de ladite ouverture (474) et/ou expansible sous l'effet d'une pression positive de manière à au moins partiellement fermer une ouverture (476) de ladite ouverture (474).

6. Un dispositif de conditionnement de pansement (312) selon la revendication 4 ou la revendication 5, dans lequel le matériau de remplissage de plaie (466) est plus compressible et/ou expansible que l'indicateur visuel (472).

7. Un dispositif de conditionnement de pansement (312) selon l'une quelconque des revendications précédentes, comprenant en outre un régulateur de pression négative (348) dans le trajet d'écoulement du fluide usé (322) et/ou le trajet d'écoulement du fluide de traitement, le régulateur de pression négative (348) comprenant une valve configurée de façon à s'ouvrir automatiquement vers l'extérieur du trajet d'écoulement afin d'augmenter la pression au niveau du pansement (414), et se fermer automatiquement pour maintenir la pression.

8. Un dispositif de conditionnement de pansement (312) selon l'une quelconque des revendications précédentes, comprenant en outre un régulateur de pression positive (360) dans le trajet d'écoulement du fluide de traitement et/ou le trajet d'écoulement du fluide usé (322), le régulateur de pression positive (360) comprenant une valve configurée de façon à pouvoir être ouverte automatiquement vers l'extérieur du trajet d'écoulement afin de diminuer la pression au niveau du pansement (314), et pour pouvoir être fermée automatiquement afin de maintenir la pression.

9. Un dispositif de conditionnement de pansement (312) selon l'une quelconque des revendications précédentes, comprenant en outre une valve sur la section de tubulure de traitement configurée de façon à empêcher le passage du fluide de traitement de l'entrée de fluide de traitement à la sortie de fluide de traitement pour permettre l'application d'une pression négative au pansement.
